# EUROPÄISCHE PATENTSCHRIFT

(11) **EP 2 576 757 B1**
(45) Veröffentlichungstag und Bekanntmachung des Hinweises auf die Patenterteilung: **16.04.2014**
(21) Anmeldenummer: 11708835.1
(22) Anmeldetag: 15.03.2011
(51) Int. Cl.: C12M 1/00, B65G 33/00, D21B 1/36, C12M 1/107, C12M 1/26, C12M 1/33, D21C 1/02

(54) **VERFAHREN UND VORRICHTUNG ZUR HYDROLYSE VON VORZUGSWEISE FESTEN, ORGANISCHEN SUBSTRATEN**
METHOD AND DEVICE FOR THE HYDROLYSIS OF PREFERABLY SOLID ORGANIC SUBSTRATES
PROCÉDÉ ET DISPOSITIF D'HYDROLYSE DE SUBSTRATS ORGANIQUES, DE PRÉFÉRENCE SOLIDES

(30) Priorität: 25.05.2010 AT 8532010
(43) Veröffentlichungstag der Anmeldung: 10.04.2013
(73) Patentinhaber: Biogas Systems GmbH, 7111 Parndorf (AT)
(72) Erfinder: DAUSER, Hermann, A-5071 Wals Siezenheim (AT)
(74) Vertreter: Babeluk, Michael
(86) Internationale Anmeldenummer: PCT/EP2011/053864
(87) Internationale Veröffentlichungsnummer: WO 2011/147601

(56) Entgegenhaltungen:
- EP-A2- 2 177 280
- WO-A2-2008/011839
- GB-A- 1 011 891
- SU-A1- 1 620 487
- US-A1- 2003 121 851

## Beschreibung

Die Erfindung betrifft ein Verfahren und eine Vorrichtung zur Hydrolyse von vorzugsweise festen, organischen Substraten, insbesondere von Energiepflanzen und pflanzlichen Abfällen, mit einem Sammelbehälter zur Aufnahme der organischen Substrate, mit einer Förderschnecke zum Transport der organischen Substrate in eine Beschickungseinrichtung zur batchweisen Befüllung eines Hydrolizers mit den organischen Substraten, wobei der Hydrolizer ausgangsseitig eine Entspannungseinrichtung mit einer ventilgesteuerten Druckblende und einem Dampfabscheider aufweist, der einem Expandertank vorgeschaltet ist.

Derartige Vorrichtungen und Verfahren dienen zur Vorbehandlung organischer Substrate, die nach der Aufbereitung in einem Hydrolizer (Einrichtung zur Thermodruckhydrolyse) einem Fermenter, beispielsweise einer Biogas- oder Biofuel-Anlage zugeführt werden.

Bei der Thermodruckhydrolyse kommt die Technologie der sogenannten "Steam-Explosion" zur Anwendung, welche beispielsweise aus Biogas- oder Biofuel-Anlagen bekannt ist. Die "Steam-Explosion" ist ein technischer Prozess, bei dem das Ausgangsmaterial auf bis zu 300°C, vorzugsweise 150°C bis 200°C aufgeheizt und unter ca. 3 bar bis 20 bar Überdruck gesetzt wird. Nachdem dieser Druck/- Temperatur-Zustand über eine bestimmte Verweilzeit eingehalten wurde, wird das Substrat spontan auf etwa Atmosphärendruck entspannt. Durch diese Schlagentspannung kommt es zur vollständigen Aufspaltung der Zellsubstanz. Die gesamte organische Substanz steht in der Folge verflüssigt für die weitere Verarbeitung zur Verfügung.

Aus der ursprünglich eingesetzten inhomogenen Substratmischung (wie beispielsweise Energiepflanzen, Ernteabfälle, etc.) wird so ein homogener Brei mit folgenden Eigenschaften:
- die Zellulose wird freigelegt;
- Inkrustierungen der Hemizellulose-Ligninkomplexe werden aufgebrochen;
- die Hemizellulose wird aufgeschlossen;
- Hefen, Schimmelpilze und andere störende Mikroorganismen werden abgetötet;
- das Substrat wird sterilisiert;
- die Faserstoffe werden destabilisiert.

Die "Steam-Explosion" übernimmt somit - vor einer weiteren Substratverarbeitung, z.B. in einer Biogasanlage - weitgehend die Verfahrensschritte Hydrolyse und Homogenisierung. Die Fermentationsbedingungen können dadurch gezielt für die Prozesse der Acido/Acetogenese und der Methanogenese optimiert werden.

Das Resultat dieser Vorbehandlung ist eine erhöhte Substratausbeute mit verbesserter Produktqualität, im Falle einer Biogasanlage z.B. eine erhöhte Substratabbaurate mit gesteigerter Gasproduktion und verbesserter Gasqualität. Typischer Weise steigt beispielsweise der spezifische Methangehalt (CH₄) an, während der schädliche Gehalt an Schwefelwasserstoff (H₂S) reduziert wird.

Die US 2003/0121851 A1 beschreibt ein Verfahren und eine Vorrichtung für die Behandlung biologisch abbaubarer organischer Abfälle. Bevor der organische Abfall der Thermodruckhydrolyse zugeführt wird, wird dem Substrat eine Lauge (KOH) zugeführt und das Substrat im Hydrolizer auf Temperaturen von 170°C bis 225°C und korrelierendem Dampfdruck unterworfen. Danach erfolgt eine Fest/- Flüssig-Separation. Das Substrat kann vor der Behandlung durch eine Dampfrückführung aus dem Hydrolizer in einem Behälter vorgeheizt werden.

Aus der WO 2008/011839 A2 ist beispielsweise eine Vorrichtung für die kontinuierliche und diskontinuierliche Hydrolyse organischer Substrate bekannt geworden. Die Anlage besteht im Wesentlichen aus einer Zerkleinerungseinrichtung für das inhomogene organische Substrat, aus welcher das Substrat in einen Zumessbehälter für den Hydrolizer gelangt. Nach der Behandlung des Substrates im Hydrolizer wird dieses in einer sogenannten "Overshooting Pipe" in einen Expandertank transportiert, aus welchem eine Abgasleitung zu einem Kondensator und eine Substratleitung zu einem Fermenter wegführen. Die Abgase werden einem Dampfkondensator zugeführt, der eine Wasserkühlung aufweist, wobei das dabei gewonnene Kondensat wieder in den Expandertank zurückgeführt wird. In der Substratleitung zum Fermenter ist ein Wärmetauscher angeordnet, dessen Abwärme über einen externen Wärmetauscherkreislauf zu einem als Vorheizeinrichtung ausgeführten Wärmetauscher führt, mit welchem das zugeführte Substrat im Bereich nach der Zerkleinerungseinrichtung erwärmt wird.

Aus der SU 1620487 A1 ist ein Hydrolizer bekannt, welcher in einem zylindrischen Gehäuse zwei konzentrisch angeordnete Förderschnecken aufweist, zwischen welchen eine Siebtrommel angeordnet ist. Das organische Material gelangt über einen Förderstutzen in einen äußeren zylindrischen Ringraum und wird mit Hilfe der ersten Förderschnecke gepresst, wobei über eine Zuleitung Heißdampf in den äußeren Ringraum zugeführt wird. Danach gelangt das Material in den inneren Hohlraum und wird dort durch die zweite Förderschnecke gegenläufig zu einer Austragsöffnung weiter transportiert.

Aus der GB 1 001 891 A1 ist eine Fördereinrichtung bekannt, mit welchem Getreide aus einem Lagerbehälter in einen Auffangbehälter transportiert werden kann, wobei gleichzeitig eine Behandlung des Getreides mit einem Fluid, beispielsweise einem Gas mit Pestiziden oder Fungiziden oder kalter bzw. heißer Luft, erfolgen kann. Die Fördereinrichtung weist eine in einem Rohr geführte archimedische Schraube auf, die auf einer drehbaren Hohlwelle angeordnet ist. Das für die Behandlung benötigte Fluid wird an einem Ende in die Hohlwelle eingeleitet und gelangt über Austrittsöffnungen in der Hohlwelle sowie der archimedischen Schraube in das transportierte Getreide, wo eine gleichmäßige Behandlung erfolgt.

Nachteilig bei den oben beschriebenen Einrichtungen und Verfahren ist, dass diese energetisch nicht optimiert sind und zumindest teilweise relativ komplex aufgebaut sind.

Schließlich ist aus der EP 2 177 280 eine Vorrichtung zur diskontinuierlichen Hydrolyse von organischen Substraten bekannt geworden, welche folgende Komponenten aufweist:
- einen mit Flüssigkeit gefüllten Vorkonditionierungstank zur Aufnahme fester, schwimmfähiger organischer Substrate mit einem Rührwerk und einer Dampfverteilereinheit, ausgeführt als spezieller Düsenstock zur Erzielung eines Flotationseffektes;
- eine Transportschnecke zur Entnahme des organischen Substrates aus einem sich oberflächlich ausbildenden Schwimmkoffer, mit einer integrierten Siebeinheit und einer Rezirkulationsleitung für den Filtratrücklauf;
- eine Beschickungseinrichtung mit einem Druckbehälter (Blow Gun) mit einer Beschickungsschleuse und einer zusätzlichen ventilgesteuerten Verbindungsleitung zum Hydrolizer;
- eine Transferpumpe zur Entnahme von Flüssigkeit aus dem Vorkonditionierungstank und Zufuhr zur Beschickungseinrichtung;
- einen Hydrolizer mit einem Rührwerk zur Durchführung der Thermodruckhydrolyse;
- eine ventilgesteuerte Entspannungseinrichtung mit einer Druckblende, einem Zyklon; sowie
- einen Expandertank mit einem integrierten Wärmetauscher.

Die aus der EP 2 177 280 bekannte Vorrichtung eignet sich u.A. besonders für die Verarbeitung von Substraten und Substratmischungen mit einem bestimmten Flüssigkeitsanteil oder Zumischung von Flüssigkeit, wobei durch Auswaschung und Aufschwimmen der schwimmfähige Festanteil vor der Beschickung des Hydrolizers abgetrennt wird. Nachteilig ist, dass durch die unkontrollierte Flüssigkeitsaufnahme des Festanteils während des Anmaischprozesses keine zuverlässige Bilanzierung der Substratzufuhr möglich ist.

Aufgabe der Erfindung ist es, eine Vorrichtung zur Hydrolyse von relativ trockenen organischen Substraten betriebstechnisch und energietechnisch zu optimieren, wobei die Anlage kompakt dimensioniert sein soll.

Diese Aufgabe wird erfindungsgemäß dadurch gelöst, dass die Förderschnecke eine Hohlwelle aufweist, die mit Heißdampf aus dem vorzugsweise als Zyklon ausgebildeten Dampfabscheider beaufschlagt ist, wobei die Hohlwelle im Förderbereich für das organische Substrat in einem Heizbereich Dampfaustrittsöffnungen zur direkten Beaufschlagung des organischen Substrats mit Heißdampf aufweist, sowie dass am Ende der Hohlwelle ein Schaltventil oder eine ventilgesteuerte Übertrittsöffnung vorgesehen ist, um durch die Dampfaustrittsöffnungen in die Hohlwelle eindringendes Substrat zu entfernen. Durch die Dampfaustrittsöffnungen in der Hohlwelle erfolgt eine effektive, gleichmäßige Bedampfung des organischen Substrats bereits vor dem Eintritt in den Hydrolizer, wobei energiesparend Abdampf aus dem Dampfabscheider verwendet wird.

Die Vorrichtung kann energetisch weiter optimiert werden, wenn der Heizbereich der Förderschnecke eine Strömungsverbindung zum Sammelbehälter für das organische Substrat aufweist, über welche aus dem Heizbereich austretender Heißdampf in den Sammelbehälter eingeleitet wird und optional in einen ggf. vorhandenen Lagerbunker übertritt.

Betriebstechnisch erfolgt die Optimierung der Vorrichtung weiters durch die bilanzierte Zuführung von Prozesswasser, wobei erfindungsgemäß in die Beschickungseinrichtung des Hydrolizers eine Zumesseinrichtung für Prozesswasser mündet, welches zur ausreichenden Wässerung des organischen Substrates vor der Einschleusung in den Hydrolizer dient. Für die Erhitzung des Prozesswassers ist ein Wärmetauscher vorgesehen, welcher in thermischem Kontakt mit dem Expandertank steht, wodurch die Abwärme des Expandertanks rückgewonnen werden kann.

Beim erfindungsgemäßen Verfahren wird somit aus den in der Thermodruckhydrolyse behandelten Substraten unmittelbar nach der Ausschleusung einer Teilmenge und deren Druckentspannung Heißdampf abgeschieden und zur Erhitzung der dem Hydrolyseverfahren zugeführten organischen Substrate eingesetzt, wobei der abgeschiedene Heißdampf direkt in eine Förderschnecke eingeblasen wird, mit welcher das organische Substrat der Thermodruckhydrolyse zugeführt wird. Der Heißdampf tritt durch Dampfaustrittsöffnungen der Hohlwelle in das organische Substrat aus, wobei durch die Dampfaustrittsöffnungen in die Hohlwelle eindringendes Substrat durch Betätigung eines endseitigen Ventils aus der Hohlwelle entfernt wird.

Erfindungsgemäß nimmt das vorerst trockene Substrat auf der Förderschnecke die Kondensationswärme des Heißdampfes auf und wird auf Temperaturen bis 70°C, vorzugsweise bis 100°C erhitzt und zusätzlich gedämpft, wodurch die Oberflächenstrukturen des Substrats aufgeweicht werden und Wasser aufgenommen wird. Durch die simultane Bewegung der Förderschnecke während des Bedampfens kann dabei der Medienkontakt intensiviert werden.

Die Erfindung wird im Folgenden anhand von schematischen Darstellungen näher erläutert. Es zeigen:
- Fig. 1: eine nicht erfindungsgemäße Vorrichtung zur Hydrolyse von vorzugsweise festen, organischen Substraten;
- Fig. 2: eine nicht erfindungsgemäße Variante der Vorrichtung gemäß Fig. 1;
- Fig. 3: ein Detail einer erfindungsgemäßen Variante der Vorrichtungen gemäß Fig. 1 und Fig. 2; sowie
- Fig. 4: eine weitere erfindungsgemäße Variante der Vorrichtung gemäß Fig. 1.

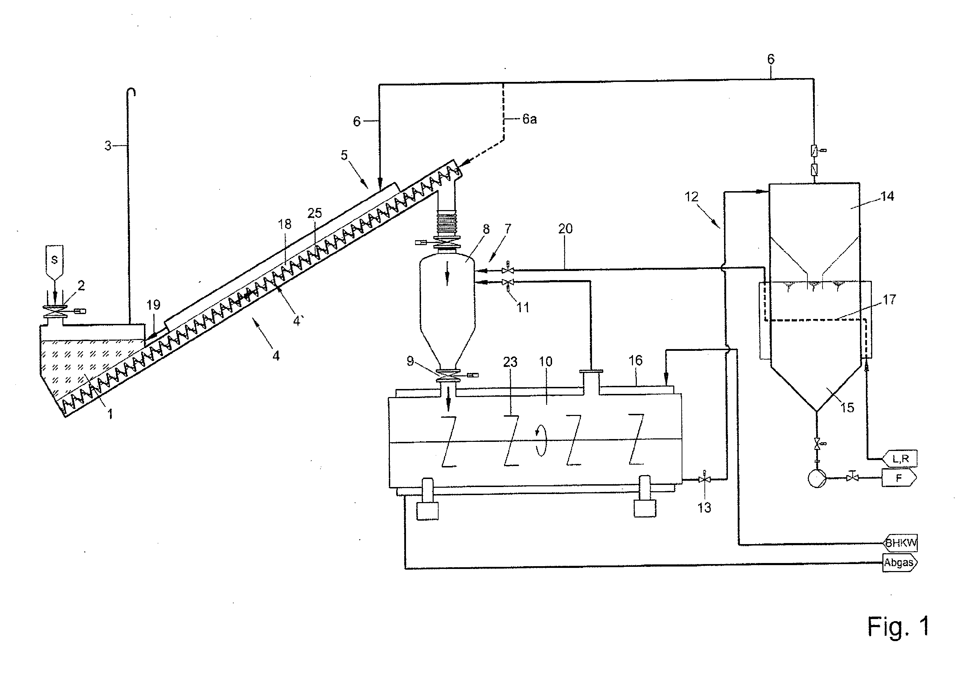

Die in Fig. 1 dargestellte Vorrichtung zur Hydrolyse von organischen Substraten besteht im Wesentlichen aus folgenden Komponenten:
- einem Sammelbehälter 1 zur Aufnahme fester organischer Substrate, beispielsweise gehäckseltes Stroh oder Silagen, mit einer Einlassöffnung 2 für das Substrat und einer Abdampfleitung 3;
- einem Fördermittel, bzw. einer Förderschnecke 4 zum Transport der organischen Substrate, mit einer Heizeinrichtung 5, welcher über eine Leitung 6 Heißdampf aus dem Dampfabscheider 14 zugeführt wird;
- einer Beschickungseinrichtung 7 mit einem Druckbehälter 8 (Blow Gun) samt ventilgesteuerter Beschickungsöffnung 9 zum Hydrolizer 10 und einer ventilgesteuerten Verbindungsleitung11 zum Hydrolizer 10;
- dem Hydrolizer 10 zur Durchführung der Thermodruckhydrolyse samt Rührwerk 23;
- einer Entspannungseinrichtung 12 mit einer ventilgesteuerten Druckblende 13, einem Dampfabscheider 14 (z.B. Zyklon) der in einen Expandertank 15 mündet;
- einer Einrichtung 16 zur Beheizung des Hydrolizers 10;
- dem Expandertank 15 mit integriertem Wärmetauscher 17.

Die Förderschnecke 4 durchsetzt einen geschlossenen Heizbereich 18 der Heizeinrichtung 5, in welchen mittels der Dampfleitung 6 Heißdampf aus dem als Zyklon ausgebildeten Dampfabscheider 14 zugeführt wird.

Weiters kann der Heizbereich 18 der Förderschnecke 4 eine Strömungsverbindung 19 zum Sammelbehälter 1 für das organische Substrat aufweisen, über welche aus dem Heizbereich 18 austretender Heißdampf in den Sammelbehälter 1 einströmt und das dort lagernde Substrat vorwärmt.

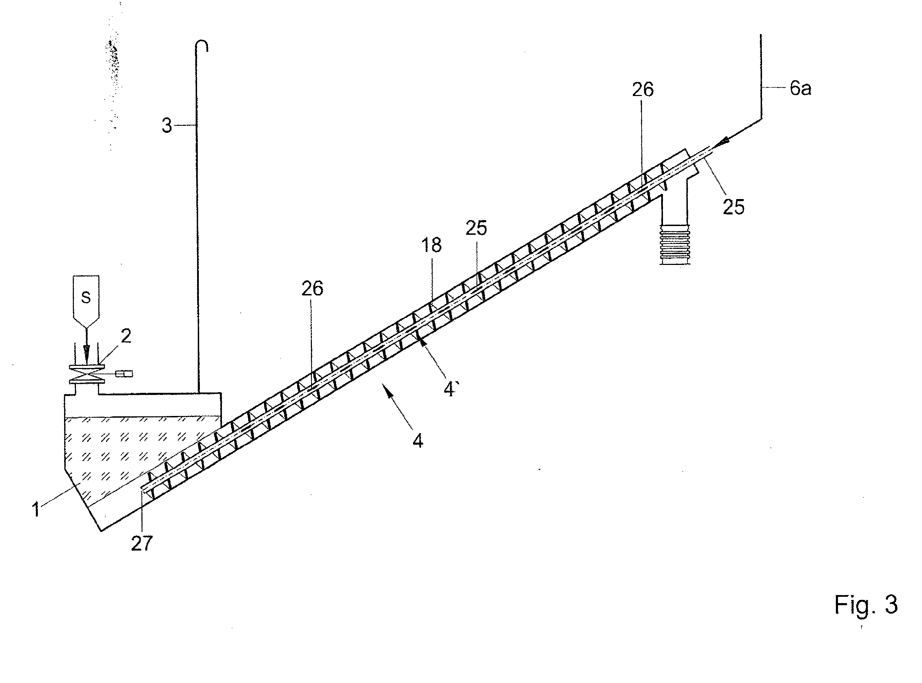

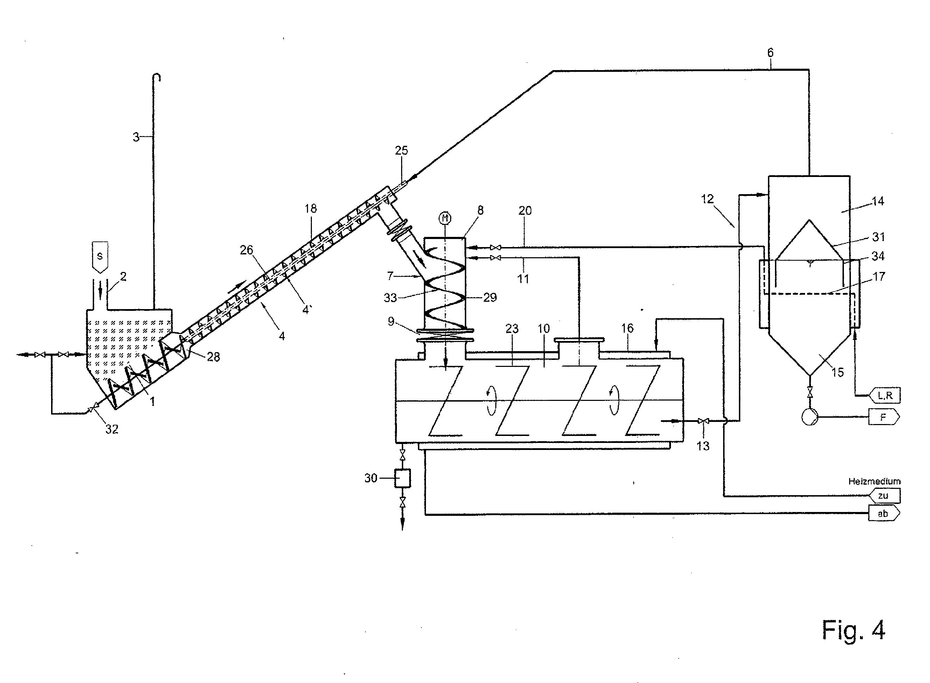

Gemäß einem in Fig. 3 dargestellten Detail der Erfindung ist die Förderschnecke 4 mit einer Hohlwelle 25 ausgestattet, die über die Leitung 6a mit Heißdampf aus dem vorzugsweise als Zyklon ausgebildeten Dampfabscheider 14 beaufschlagt ist. Der Heißdampf kann der Hohlwelle 25 auch direkt mittels Dampfleitung 6 zugeführt werden (siehe Fig. 4). Die Hohlwelle 25 weist im Förderbereich für das organische Substrat beispielsweise schlitzförmige Dampfaustrittsöffnungen 26 auf, über welche eine effektive, gleichmäßige Bedampfung des Substrates erfolgt.

An dem in den Sammelbehälter 1 eintauchenden Ende weist die Hohlwelle 25 der Förderschnecke 4 eine vorzugsweise ventilgesteuerte Übertrittsöffnung 27 zur Abgabe von überschüssigem Heißdampf in den Sammelbehälter 1 auf.

### Verfahrensbeschreibung :

Das Substrat, das im Sammelbehälter 1 vorliegt ist üblicherweise kurzfaseriges oder krümeliges Material mit einer Partikelgröße bis zu 5 cm, typisch mit einer Trockensubstanz von ca. 30% (z.B. Silagen) bis zu 90% (z.B. Stroh).

Die Förderschnecke 4 entnimmt das Substrat aus dem Sammelbehälter 1 und fördert es zur Beschickungseinrichtung 7 des Hydrolizers 10. (Die Substratmenge, die sich bei typischem Füllgrad in der Förderschnecke 4 befindet, entspricht dabei praktischerweise in etwa einer Beschickungscharge des Hydrolizers 10, ebenso einer Füllung des Sammelbehälters 1).

Sammelbehälter 1 und Förderschnecke 4 sind derart gestaltet, dass über eine Verteiler- und Zuführeinrichtung der Heizeinrichtung 5, insbesondere den Dampfaustrittsöffnungen in der Hohlwelle 25, Heißdampf aus dem Entspannungsprozess der Entspannungseinrichtung 12 direkt zum dort befindlichen Substrat geführt werden kann. Durch die beim Kontakt des Dampfes mit dem Substrat abgegebene Kondensationswärme erfolgt eine Erhitzung des Substrates bis auf 100°C, typisch auf mehr als 70°C. Dadurch reduziert sich der Heizaufwand zum Erreichen des Betriebspunktes von bis zu 180°C im Hydrolizer 10 beträchtlich.

Ein zusätzlicher, positiver Effekt ist die Dämpfung des Substrates, d.h. die Aufweichung von Oberflächenstrukturen und gleichzeitig die Aufnahme von Wasser durch das Substrat. Die feuchte Luft bzw. der Restdampf entweichen aus dem Heizbereich 18 entweder in den Sammelbehälter 1 oder werden als Abluft ausgeschleust.

Die Förderschnecke 4 überführt sequentiell eine definierte Menge an vorgewärmtem und befeuchtetem Substrat in den Druckbehälter 8 der Beschickungseinrichtung 7. Nach dem Erreichen der erforderlichen Substratfüllung wird zur Herstellung einer ausreichend gewässerten Substratmischung zusätzlich Prozesswasser über eine Zumesseinrichtung 20 in definierter Menge in den Druckbehälter 8 eingebracht. Dieses Prozesswasser wird davor zur Reduktion der im Hydrolizer 10 erforderlichen Erhitzung über einen Wärmeaustauscher 17 im Expandertank 15 auf 50°C bis 100°C vorgewärmt.

Diese Art der sequentiellen Befüllung ermöglicht es, eine genaue und für das Substrat und das Prozesswasser getrennte Bilanzierung der dem Hydrolizer 10 zugeführten Massenströme durchzuführen. Dadurch ist eine gezielte Steuerung des Systemdurchsatzes und der Betriebsparameter möglich.

Der Druckbehälter 8 der Beschickungseinrichtung 7 ist eine sogenannte "Blow Gun", d.h. der Behälters wird nach der Füllung mit einer Charge durch Schließen der Einlassöffnung zur Atmosphäre druckdicht verschlossen, und durch Öffnung einer ventilgesteuerten Verbindungsleitung 11 auf den gleichen Systemdruck wie der Hydrolizer 10 gebracht. Anschließend wird das Ventil der Verbindungsleitung 11 wieder verschlossen.

Die Entleerung des Druckbehälters 8 erfolgt zyklisch durch die ventilgesteuerte Beschickungsöffnung 9 nach dem teilweisen Entleeren des Hydrolizers 10 durch die Druckdifferenz zwischen Druckbehälter 8 und Hydrolizer 10 (in der Regel 1 bis 2 bar Differenzdruck). Falls erforderlich, kann der Systemdruck zusätzlich z.B. durch Zugabe von Druckluft in den Druckbehälter 8 erhöht werden, um eine vollständige Entleerung der Beschickungseinrichtung 7 zu gewährleisten.

Nach der Befüllung des Hydrolizers 10 aus der "Blow Gun" verläuft der Hydrolisierungsprozess durch kontinuierliche Aufheizung über eine Heizeinrichtung 16 und simultane Druckerhöhung über eine bestimmte Verweildauer von z.B. 30 Minuten bis zu mehreren Stunden.

Im Anschluss wird ein definiertes Volumen durch den Systemüberdruck ausgeschleust, und durch die spontane Entspannung in der Entspannungseinrichtung 12 verbunden mit einem Druckschlag desintegriert.

Die Beschickung und Ausschleusung von Substrat in und aus dem Hydrolizer 10 erfolgt dabei in kurzer Zyklenfolge, z.B. 2 bis 4 Zyklen pro Stunde, und umfasst dabei nur einen Teil des Hydrolizer-Volumens, beispielsweise 10% bis 30%. Diese besondere Betriebsweise mit einer schnellen Abfolge von Beschickungs- und Entnahmezyklen für einen Teil des Reaktorvolumens wird in der Folge ais quasikontinuierlich bezeichnet.

Die quasikontinuierliche Betriebsweise ergibt mehrere entscheidende Vorteile gegenüber bekannten kontinuierlichen und diskontinuierlichen Prozessen:
a) Durch den stoßweisen Ablass können bei der Druckblende 13 große Blendenweiten mit hohem Durchsatz verwendet werden, wodurch Blendenverschleiß und Verstopfungen, wie sie typisch für kontinuierliche Verfahren sind, nicht auftreten.
b) Durch die Entnahme nur eines Teiles des Hydrolizer-Volumens wird das gesamte Substrat mit dem maximalen Entspannungseffekt, dem sogenannten "Schärfegrad", ausgeschleust, wodurch ein optimales Desintegrationsergebnis erzielt wird. Klassische diskontinuierliche Batchverfahren mit bei jedem Zyklus kompletter Reaktorentleerung haben einen unvermeidbaren Schlupf an gering desintegriertem Substrat, da mit fortschreitender Reaktorentleerung der für den Treibeffekt vorliegende Systemüberdruck kontinuierlich sinkt.
c) Klassische Batchverfahren weisen aufgrund der Betriebsweise einen zyklischen Bedarf an Beheizung auf, so dass eine erhöhte Spitzenleistung sowie ein diskontinuierlicher Verbrauch an Heizmedium zu bedienen ist. Durch die quasikontinuierliche Betriebsweise des Hydrolizers 10 kann die Beheizung permanent mit gleicher Leistung erfolgen, was z.B. der typischen Betriebsweise einer Biogasanlage entgegenkommt.

Die Beheizung des Hydrolizers 10 erfolgt üblicherweise durch Dampf, Thermoöl oder einen Gasbrenner. Im Falle einer Kombination des Systems mit einer Biogasanlage mit Co-Generation (Erzeugung von Elektrizität und Abwärme durch ein Blockheizkraftwerk BHKW oder eine gleichartige Verbrennungsmaschine) als typischer Anlagenkonfiguration kann eine Einrichtung zur Zufuhr heißer Abgase der Co-Generation zur Direktbeheizung des Hydrolizers 10 verwendet werden. Diese Betriebsweise führt zu einer weiteren energetischen Systemoptimierung.

Das nach dem Austritt aus dem Hydrolizer 10 weitgehend desintegrierte bzw. verflüssigte Substrat gelangt in einen Zyklon 14, wobei ein Gasanteil (Heißdampf) abgeschieden wird, und der flüssig/feste Anteil nach unten in den Expandertank 15 abfließt.

Durch einen als Doppelmantel oder Register ausgeführten Wärmetauscher 17 im Expandertank 15 kann die hohe Systemtemperatur des Substrates (ca. 100°C) genutzt werden, um z.B. das im Druckbehälter 8 zur Flüssigkeitsanreicherung eingesetzte Prozesswasser vorzuheizen.

Aus dem Expandertank 15 wird das behandelte Substrat durch eine geeignete Fördereinrichtung (z.B. eine Dickstoffpumpe) zur weiteren Verarbeitung überführt.

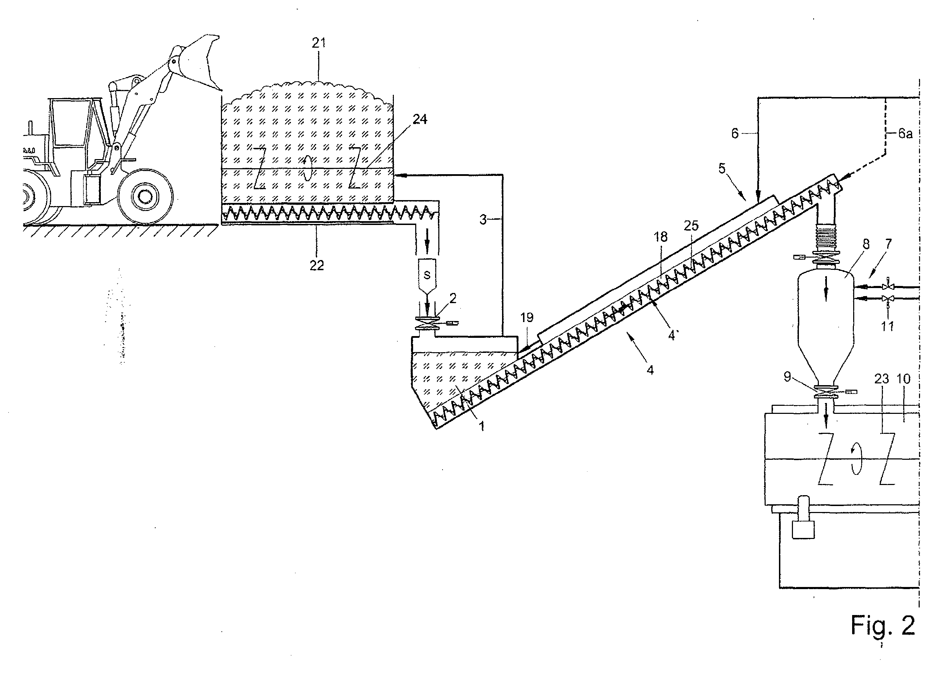

Bei der in Fig. 2 dargestellten Ausführungsvariante ist dem Sammelbehälter 1 zur Aufnahme der organischen Substrate ein Lagerbunker 21 mit einem Mischer 24 und einem Transportförderer 22 vorgeschaltet. Der Mischer 24 dient zur Zerstörung von Substrat-Brücken, die ein Nachsacken des Substrates in den Transportförderer 22 verhindern.. Durch die Rotationsbewegung des Mischers 24 kann auch die Zuführung des Substrates zum Förderer optimiert werden.

Durch eine Umleitung der Abdampfleitung 3 aus dem Sammelbehälter 1 in den Lagerbunker 21 kann der restliche Dampf nochmals zur Vorwärmung des Substrates eingesetzt werden. Zusätzlich lockert der Druckstoß beim Dampfeintritt das Substrat im Bunker auf, was dabei hilft, Brückenbildungen im Substrat zu vermeiden.

Bei der Ausführungsvariante gemäß Fig. 4 weist die mit den Dampfaustrittsöffnungen 26 versehene Hohlwelle 25 der Förderschnecke 4' am unteren Ende der Welle ein Schaltventil 32 auf, durch das flüssiges und festes Substrat, das durch die Dampfaustrittsöffnungen 26 in die Hohlwelle 25 gelangen kann, aus dieser wieder entfernt wird. Dies geschieht in der Regel zyklisch mit der Zufuhr des aus dem Dampfabscheider 14 über die Verbindung 6 zugeleiteten recycelten Abdampfes. Durch dessen Überdruck werden Stoffe, die sich im Inneren der Hohlwelle 25 ablagern, durch das geöffnete Schaltventil 32 ausgeblasen, und wahlweise in den Sammelbehälter 1, den hier nicht dargestellten Lagerbunker 21 (siehe Fig. 2) oder eine andere Sammeleinrichtung überführt. Durch diese Maßnahme wird eine Verstopfung der Hohlwelle 25 bzw. deren Dampfaustrittsöffnungen 26 durch Substratteile verhindert.

Weiters kann mit Hilfe des Schaltventils 32 auch eine Rückspülung mit flüssigen Reinigungsmedien oder Druckluft durchgeführt werden. Zusätzlich kann das Schaltventil 32 dazu genutzt werden, um einen Überschuss an Prozessdampf in den Sammelbehälter 1 oder den Lagerbunker 21 einzuleiten, und einen Systemüberdruck im Heizbereich 18 abzubauen.

Lose Substrate, wie zerkleinertes Stroh und Silagen, weisen unter Umständen eine sehr geringe Schüttdichte auf, was in der Förderschnecke 4 bzw. dem Heizbereich 18 dazu führt, dass keine ausreichende Substratmenge für eine komplette Batch-Füllung der Beschickungseinrichtung 7 erreicht wird, und die angestrebte Vollfüllung des rohrförmigen Heizbereiches 18 verfehlt wird.

Um dies zu vermeiden, weist die Förderschnecke 4' im Bereich des Sammelbehälters 1 einen größeren Durchmesser auf als im Heizbereich 18, so dass am Übergang in den Heizbereich 18 eine Kompaktierungszone 28 entsteht, in der das geförderte Material verdichtet wird. Der Schneckengang der Förderschnecke wird beispielsweise derart variiert, dass der Durchmesser der Schnecke am Übergang in den Heizbereich 18 im Verhältnis 2:1 reduziert wird, wodurch der Schneckengang im Heizbereich kompakt gefüllt wird. Durch diesen Kompaktierungsvorgang wird kein Überdruck aufgebaut, er dient lediglich der Erhöhung der Substratdichte.

Praxisversuche haben gezeigt, dass beim Einsatz von "sperrigen", fasrigen Substraten im Druckbehälter 8 (Blow Gun) der Beschickungseinrichtung 7, insbesondere bei geringen Austauschraten, der durch den Druckabgleich zwischen Hydrolizer 10 und Blow Gun 8 vor dem Ausschleusen erzeugte Druckausgleich nicht zuverlässig ausreicht, um eine schnelle und vollständige Entleerung der Blow Gun sicher zu stellen.

Dies kann dadurch gelöst werden, dass im Druckbehälter 8 der Beschickungseinrichtung 7 eine drehbare Räumschnecke 29 angeordnet ist, die ein den Füllungsvorgang nicht behinderndes, schmales spiralförmiges Metallband 33 aufweist, das der Innenwand des Druckbehälters 8 zugeordnet ist. Die Räumschnecke 29 wird während des Beschickungsvorganges des Hydrolizers 10 mit Förderrichtung nach unten in Drehbewegung versetzt und sorgt dafür, dass im gesamten Wandbereich des Druckbehälters 8 anhaftendes Substrat abgelöst wird, so dass auch mit geringem Systemüberdruck eine zügige Abwärtsbewegung entsteht, die eine schnelle und vollständige Entleerung der Blow Gun sicher stellt. Die Räumschnecke 29 baut selbst keinen Überdruck auf, ist also keine sogenannte Stopfschnecke, wodurch die Installation verschleißarm und betriebssicher ausgeführt werden kann.

Durch die meist landwirtschaftliche Herkunft der verarbeiteten Substrate lässt es sich nicht vermeiden, dass schwere Fremdkörper, wie Steine oder auch kleine Metallteile, in das System eingetragen werden. Da die Gesamtanordnung bevorzugt auf vorgeschaltete Siebe oder Abscheider verzichtet, sammeln sich diese Stoffe im Hydrolizer 10 mit der Zeit an, da sie aufgrund der quasi-kontinuierlichen Teilbefüllung bzw. Teilentnahme und eines üblicherweise nicht in Bodennähe des Hydrolizers 10 angeordneten Anschlusses der Entspannungseinrichtung 12 nicht entweichen können.

Um die Ausbildung eines Beschädigungen verursachenden Bodensatzes zu verhindern, ist ein effektives Austragssystem für derartige Fremdkörper vorgesehen. Der Hydrolizer 10 ist bevorzugt über eine Ventilschleuse mit einer Sedimentkammer 30 verbunden, die während des Räumvorganges geöffnet und anschlieβend wieder geschlossen wird. Nach einem Druckausgleich zur Atmosphäre kann die Sedimentkammer 30 durch eine zweite Ventilschleuse entleert werden. Auf diese Weise ist eine Entnahme von abgelagerten Fremdkörpern im laufenden Betrieb möglich.

Die hohe Temperatur des aus dem Hydrolizer 10 ausgeschleusten und in den Zyklon bzw. Dampfabscheider 14 eingeleiteten Substrates wird genutzt, um Prozesswasser oder andere Flüssigkeiten vorzuheizen. Der Wärmeübergang im Bereich des Wärmetauschers 17 kann dadurch optimiert werden, dass das vom Zyklon 14 in den Expandertank 15 übertretende, heiße Substrat gezielt an die Wärmeübergangsfläche, in diesem Fall vorzugsweise die Behälterwand, herangeführt wird.

Das wird vorzugsweise dadurch erreicht, dass der Dampfabscheider 14 einen Einbaukegel 31 aufweist, an welchen ggf. ein zylindrischer Bereich 34 anschließt, welcher mit der Behälterwand einen Ringspalt bildet, wobei die Entspannungseinrichtung 12 tangential in den Dampfabscheider 14 einmündet. Der für einen Zyklon typische Aufbau des umgedrehten Kegelspitzes mit zentralem Ablauf (siehe Fig. 1) wird hier invertiert, so dass der Substratablauf im Ringspalt am Außenumfang des Einbaukegels 31 erfolgt. Dies korreliert gut mit der tangentialen Zyklon-Beschickung, die zu einer umlaufenden Beaufschlagung der Zyklonwand mit flüssigem Substrat führt. Das heiße Substrat fließt nun direkt an der Heizfläche des Wärmetauschers 17 entlang, bevor es sich mit dem restlichen Material im Expandertank 15 vermischt.

## Patentansprüche

1. Vorrichtung zur Hydrolyse von vorzugsweise festen, organischen Substraten, insbesondere von Energiepflanzen und pflanzlichen Abfällen, mit einem Sammelbehälter (1) zur Aufnahme der organischen Substrate, mit einer Förderschnecke (4') zum Transport der organischen Substrate in eine Beschickungseinrichtung (7) zur batchweisen Befüllung eines Hydrolizers (10) mit den organischen Substraten, wobei der Hydrolizer (10) ausgangsseitig eine Entspannungseinrichtung (12) mit einer ventilgesteuerten Druckblende (13) und einem Dampfabscheider (14) aufweist, der einem Expandertank (15) vorgeschaltet ist, **dadurch gekennzeichnet, dass** die Förderschnecke (4') eine Hohlwelle (25) aufweist, die mit Heißdampf aus dem vorzugsweise als Zyklon ausgebildeten Dampfabscheider (14) beaufschlagt ist, wobei die Hohlwelle (25) im Förderbereich für das organische Substrat in einem Heizbereich (18) Dampfaustrittsöffnungen (26) zur direkten Beaufschlagung des organischen Substrats mit Heißdampf aufweist, sowie dass am Ende der Hohlwelle (25) eine ventilgesteuerte Übertrittsöffnung (27) oder ein Schaltventil (32) vorgesehen ist, um durch die Dampfaustrittsöffnungen (26) in die Hohlwelle (25) eindringendes Substrat zu entfernen.

2. Vorrichtung nach Anspruch 1, **dadurch gekennzeichnet, dass** die ventilgesteuerte Übertrittsöffnung (27) zur Abgabe von Heißdampf direkt in den Sammelbehälter (1) an dem in den Sammelbehälter (1) eintauchenden Ende der Hohlwelle (25) angeordnet ist.

3. Vorrichtung nach Anspruch 1 oder 2, **dadurch gekennzeichnet, dass** der Heizbereich (18) der Förderschnecke (4') eine Strömungsverbindung (19) zum Sammelbehälter (1) für das organische Substrat aufweist, über welche aus dem Heizbereich (18) austretender Heißdampf in den Sammelbehälter (1) einströmt.

4. Vorrichtung nach einem der Ansprüche 1 bis 3, **dadurch gekennzeichnet, dass** die Förderschnecke (4') im Bereich des Sammelbehälters (1) einen größeren Durchmesser aufweist als im Heizbereich (18), so dass am Übergang in den Heizbereich (18) eine Kompaktierungszone (28) entsteht, in der das geförderte organische Substrat verdichtet wird.

5. Vorrichtung nach einem der Ansprüche 1 bis 4, **dadurch gekennzeichnet, dass** in die Beschickungseinrichtung (7) des Hydrolizers (10) eine Zumesseinrichtung (20) für Prozesswasser mündet, welches zur ausreichenden Wässerung des organischen Substrates vor der Einschleusung in den Hydrolizer (10) dient.

6. Vorrichtung nach Anspruch 5, **dadurch gekennzeichnet, dass** für die Erhitzung des Prozesswassers ein Wärmetauscher (17) vorgesehen ist, welcher in thermischem Kontakt mit dem Expandertank (15) steht.

7. Vorrichtung nach einem der Ansprüche 1 bis 6, **dadurch gekennzeichnet, dass** der Dampfabscheider (14) einen Einbaukegel (31) aufweist, an welchen ggf. ein zylindrischer Bereich (33) anschließt, der mit der Behälterwand einen Ringspalt bildet, wobei die Entspannungseinrichtung (12) tangential in den Dampfabscheider (14) einmündet.

8. Vorrichtung nach einem der Ansprüche 1 bis 7, **dadurch gekennzeichnet, dass** die Beschickungseinrichtung (7) zur Befüllung des Hydrolizers (10) einen Druckbehälter (8) aufweist, der neben einer ventilgesteuerten Beschickungsöffnung (9) zum Hydrolizer (10) eine ventilgesteuerte Verbindungsleitung (11) zur Herstellung eines Temperatur- und Druckausgleichs mit dem Hydrolizer (10) aufweist.

9. Vorrichtung nach Anspruch 8, **dadurch gekennzeichnet, dass** im Druckbehälter (8) der Beschickungseinrichtung (7) eine drehbare Räumschnecke (29) angeordnet ist, die ein den Füllungsvorgang nicht behinderndes, schmales spiralförmiges Metallband (33) aufweist, das der Innenwand des Druckbehälters (8) zugeordnet ist.

10. Vorrichtung nach einem der Ansprüche 1 bis 9, **dadurch gekennzeichnet, dass** der Hydrolizer (10) über eine Ventilschleuse mit einer Sedimentkammer (30) verbunden ist, welche zur Aufnahme von aus dem Hydrolizer (10) ausgeschleusten Fremdkörpern dient.

11. Vorrichtung nach einem der Ansprüche 1 bis 10, **dadurch gekennzeichnet, dass** dem Sammelbehälter (1) zur Aufnahme der organischen Substrate ein Lagerbunker (21) mit einem Transportförderer (22) vorgeschaltet ist, wobei eine Abdampfleitung (3) aus dem Sammelbehälter (1) in den Lagerbunker (21) geführt ist.

12. Verfahren zur Hydrolyse von vorzugsweise festen, organischen Substraten, insbesondere von Energiepflanzen, sowie pflanzlichen Abfällen, mittels Thermodruckhydrolyse (Steam-Explosion), wobei aus den in der Thermodruckhydrolyse behandelten Substraten unmittelbar nach dem Ausschleusen einer Teilmenge des Substrats durch Druckentspannung Heißdampf abgeschieden wird und zur Erhitzung der dem Hydrolyseverfahren zugeführten organischen Substrate eingesetzt wird, **dadurch gekennzeichnet, dass** der abgeschiedene Heißdampf direkt in die Hohlwelle (25) einer Förderschnecke (4') eingeblasen wird, mit welcher das organische Substrat der Thermodruckhydrolyse zugeführt wird, wobei der Heißdampf durch Dampfaustrittsöffnungen (26) der Hohlwelle (25) in das organische Substrat austritt und durch die Dampfaustrittsöffnungen (26) in die Hohlwelle (25) eindringendes Substrat durch Betätigung eines endseitigen Ventils (32) aus der Hohlwelle (25) entfernt wird.

13. Verfahren nach Anspruch 12, **dadurch gekennzeichnet, dass** das vorerst trockene Substrat auf der Förderschnecke (4') Kondensationswärme des Heißdampfes aufnimmt auf Temperaturen bis 70°C, vorzugsweise bis 100°C erhitzt und gedämpft wird.

14. Verfahren nach Anspruch 12 oder 13, **dadurch gekennzeichnet, dass** ein Teil der bei der Druckentspannung des Substrats anfallenden Wärmeenergie zur Vorwärmung von Prozesswasser verwendet wird, das dem Substrat vor der Thermodruckhydrolyse in definierter Menge zugemessen wird.

## Claims

1. Apparatus for the hydrolysis of preferably solid, organic substrates, especially energy crops and vegetable waste, which comprises a collecting bin (1) for receiving organic substrates and a screw conveyor (4') for transporting the organic substrates to a charging device (7) for batchwise filling of a hydrolizer (10) with the organic substrates, the hydrolizer (10) being provided on the output side with a depressurization unit (12) with a valve-controlled pressure baffle (13) and a steam separator (14) upstream of a flash tank (15), **characterised in that** the screw conveyor (4') is provided with a hollow shaft (25), into which is fed superheated steam from the steam separator (14) preferably configured as a cyclone, the hollow shaft (25) having steam vents (26) in a heating zone (18) in the conveying area for the organic substrate, for directly subjecting the organic substrate to superheated steam, and that at the end of the hollow shaft (25) a valve-controlled exit opening (27) or a switching valve (32) is provided for removing any substrate entering the hollow shaft (25) via the steam vents (26).

2. Apparatus according to claim 1, **characterised in that** the valve-controlled exit opening (27) is positioned at the end of the hollow shaft (25) dipping into the collecting bin (1), such that superheated steam is directly fed into the collecting bin (1).

3. Apparatus according to claim 1 or 2, **characterised in that** the heating zone (18) of the screw conveyor (4') has a flow-connection (19) to the collecting bin (1) for the organic substrate, through which superheated steam exiting the heating zone (18) flows into the collecting bin (1).

4. Apparatus according to any of claims 1 to 3, **characterised in that** the screw conveyor (4') has a larger diameter in the area of the collecting bin (1) than in the heating zone (18), resulting in a compactification zone (28) at the transition to the heating zone (18), in which the transported organic substrate is compacted.

5. Apparatus according to any of claims 1 to 4, **characterised in that** a metering unit (20) for process water opens into the charging unit (7) of the hydrolizer (10), which serves for sufficient watering of the organic substrate prior to its entry into the hydrolizer (10).

6. Apparatus according to claim 5, **characterised in that** for heating of the process water a heat exchanger (17) is provided, which is in thermal contact with the flash tank (15).

7. Apparatus according to any of claims 1 to 6, **characterised in that** the steam separator (14) has an internal cone (31) with possibly an adjoining cylindrical area (34), which forms an annular gap with the container wall, with the depressurization unit (12) entering the steam separator (14) tangentially.

8. Apparatus according to any of claims 1 to 7, **characterised in that** the charging unit (7) for filling the hydrolizer (10) has a pressure vessel (8), which is provided, besides a valve-controlled charging port (9) into the hydrolizer (10), with a valve-controlled connecting line (11) for temperature and pressure equalization with the hydrolizer (10).

9. Apparatus according to claim 8, **characterised in that** a rotatable clearing screw (29) is disposed in the pressure vessel (8) of the charging unit (7), which has the form of a narrow helical metal strip (33) along the inner wall of pressure vessel (8) and will not impede the filling process.

10. Apparatus according to any of claims 1 to 9, **characterised in that** the hydrolizer (10) is connected via a valve to a sediment chamber (30), which receives foreign objects removed from the hydrolizer (10).

11. Apparatus according to any of claims 1 to 10, **characterised in that** upstream of the collecting bin (1) for receiving organic substrates there is a storage bunker (21) with a conveyor (22), a waste steam line (3) leading from the collecting bin (1) to the storage bunker (21).

12. Method for hydrolysis of preferably solid, organic substrates, in particular energy crops and vegetable waste, by means of thermal pressure hydrolysis (steam explosion), where from the substrates treated by thermal pressure hydrolysis superheated steam is separated by flashing immediately after discharge of part of the substrate from the hydrolizer and is used for heating the organic substrates fed to the process of hydrolysis, **characterised in that** the superheated steam separated is blown directly into the hollow shaft (25) of a screw conveyor (4'), which transports the organic substrate to the thermal pressure hydrolysis reactor, the superheated steam entering the organic substrate via steam vents (26) in the hollow shaft (25), and substrate penetrating into the hollow shaft (25) via the steam vents (26) being removed from the hollow shaft (25) by actuating a valve (32) at the end of said shaft.

13. Method according to claim 12, **characterised in that** the initially dry substrate receives condensation heat from the superheated steam while being on the screw conveyor (4') and is steamed and heated to 70°C, preferably 100°C.

14. Method according to claim 12 or 13, **characterised in that** part of the heat energy stemming from the flashing of the substrate is used to preheat process water, a defined amount of which is added to the substrate prior to thermal pressure hydrolysis.

## Revendications

1. Dispositif d'hydrolyse de substrats organiques de préférence solides en particulier de plantes énergétiques et de déchets végétaux comportant un réservoir collecteur (1) pour la réception des substrats organiques, une vis transporteuse (4') pour le transport des substrats organiques dans un dispositif de chargement (7) permettant de remplir de façon discontinue une cuve d'hydrolyse (10) avec les substrats organiques, cette cuve d'hydrolyse (10) comportant côté sortie un dispositif de détente (12) équipé d'un diaphragme de pression commandé par soupape (13) et d'un séparateur de vapeur (14) qui est monté en amont d'un réservoir d'expansion (15),
**caractérisé en ce que**
la vis transporteuse (4') comporte un arbre creux (25) qui est alimenté avec de la vapeur chaude provenant du séparateur de vapeur (14) qui est de préférence réalisé sous la forme d'un cyclone, l'arbre creux (25) comportant, dans la zone d'alimentation du substrat organique, dans une zone chaude (18), des ouvertures de sortie de vapeur (26) permettant une alimentation directe du substrat organique avec de la vapeur chaude, et, à l'extrémité de l'arbre creux (25) il est prévu une ouverture de commutation (27) commandée par soupape ou une soupape de commutation (32) pour permettre d'éliminer le substrat pénétrant dans l'arbre creux (25) par les ouverture de sortie de vapeur (26).

2. Dispositif conforme à la revendication 1,
**caractérisé en ce que**
l'ouverture de commutation (27) commandée par soupape est située à l'extrémité de l'arbre creux (25) plongeant dans le réservoir collecteur (1) pour permettre la distribution de vapeur chaude directement dans ce réservoir collecteur (1).

3. Dispositif conforme à la revendication 1 ou 2,
**caractérisé en ce que**
la zone chaude (18) de la vis transporteuse (4') comporte une liaison d'écoulement (19) vers le réservoir collecteur (1) du substrat organique par l'intermédiaire de laquelle la vapeur chaude sortant de la zone chaude (18) s'écoule dans le réservoir collecteur (1).

4. Dispositif conforme à l'une des revendications 1 à 3,
**caractérisé en ce que**
la vis transporteuse (4') a dans la zone du réservoir collecteur (1) d'un plus grand diamètre que dans la zone chaude (18) de façon à former au niveau de la transition dans la zone chaude (18) une zone de compactage (28) dans laquelle le substrat organique fourni est comprimé.

5. Dispositif conforme à l'une des revendications 1 à 4,
**caractérisé en ce que**
dans le dispositif de chargement (7) de la cuve d'hydrolyse (10) débouche un dispositif de dosage (20) de l'eau de procédé qui sert à obtenir un hydratation suffisante du substrat organique avant son introduction dans la cuve d'hydrolyse (10).

6. Dispositif conforme à la revendication 5,
**caractérisé en ce que**
pour permettre le chauffage de l'eau de procédé, il est prévu un échangeur de chaleur (17) qui est en contact thermique avec le réservoir d'expansion (15).

7. Dispositif conforme à l'une des revendications là 6,
**caractérisé en ce que**
le séparateur de vapeur (14) comporte un cône de montage (31) auquel se raccorde le cas échéant une zone cylindrique (33) qui définit un passage annulaire avec la paroi du réservoir, le dispositif de détente (12) débouchant tangentiellement dans le séparateur de vapeur (14).

8. Dispositif conforme à l'une des revendications 1 à 7,
**caractérisé en ce que**
le dispositif de chargement (7) comporte pour permettre de remplir la cuve d'hydrolyse (10) un réservoir sous pression (8) qui comporte outre une ouverture de chargement (9) vers la cuve d'hydrolyse (10) commandée par soupape une conduite de liaison (11) commandée par soupape permettant d'obtenir un équilibrage de pression et de température avec la cuve d'hydrolyse (10).

9. Dispositif conforme à la revendication 8,
**caractérisé en ce que**
dans le réservoir sous pression (8) du dispositif de chargement (7) est montée une vrille mobile en rotation (29) qui comporte une petite bande métallique en forme de spirale (34) n'entravant pas le processus de remplissage qui est associé à la paroi interne du réservoir sous pression (8).

10. Dispositif conforme à l'une des revendications 1 à 9,
**caractérisé en ce que**
la cuve d'hydrolyse (10) est reliée par l'intermédiaire d'une écluse de soupape à une chambre de sédimentation (30) qui sert à la réception de corps étrangers extraits de la cuve d'hydrolyse (10).

11. Dispositif conforme à l'une des revendications 1 à 10,
**caractérisé en ce qu'**
en amont du réservoir collecteur (1) est monté un silo de stockage (21) équipé d'un convoyeur de transport (22) pour la réception des substrats organiques, une conduite d'évacuation de la vapeur (3) étant montée du réservoir collecteur (1) vers le silo de stockage (21).

12. Procédé d'hydrolyse de substrats organiques de préférence solides en particulier de plantes énergétiques ou de déchets végétaux par hydrolyse thermique sous pression (Steam-Explosion), selon lequel de la vapeur chaude est extraite des substrats traités par hydrolyse thermique sous pression, immédiatement après l'évacuation d'une partie de ces substrats, et est utilisée pour chauffer les substrats organiques soumis au procédé d'hydrolyse,
**caractérisé en ce que**
la vapeur chaude extraite est directement insufflée dans l'arbre creux (25) d'une vis transporteuse (4') par laquelle le substrat organique est transféré vers l'hydrolyse thermique sous pression, la vapeur chaude s'évacuant par des ouvertures de sortie de vapeur (26) de l'arbre creux (25) dans le substrat organique et, le substrats pénétrant dans l'arbre creux (25) par les ouvertures de sortie de vapeur (26), étant extrait de l'arbre creux (25) par actionnement d'une soupape (32) située à son extrémité.

13. Procédé conforme à la revendication 12,
**caractérisé en ce que**
le substrat préalablement sec recueille sur la vis transporteuse (4') de la chaleur de condensation de la vapeur d'eau et est chauffé et vaporisé à des températures allant jusqu'à 70°C de préférence jusqu'à 100°C.

14. Procédé conforme à la revendication 12 ou 13,
**caractérisé en ce que**
une partie de l'énergie thermique produite lors de la détente du substrat est utilisée pour préchauffer l'eau de procédé qui est transférée en étant dosée en quantité définie dans le substrat avant l'hydrolyse thermique sous pression.
